Europäisches Patentamt

European Patent Office

Office européen des brevets

(19)

(11) Numéro de publication : **0 442 774 A1**

(12) **DEMANDE DE BREVET EUROPEEN**

(21) Numéro de dépôt : 91400239.9

(22) Date de dépôt : 31.01.91

(51) Int. Cl.⁵ : **A61B 5/0416, A61B 5/0428**

Le titre de l'invention a été modifié (Directives relatives à l'examen pratiqué à l'OEB, A-III, 7.3)

(30) Priorité : 31.01.90 FR 9001147
10.07.90 FR 9008769

(43) Date de publication de la demande :
21.08.91 Bulletin 91/34

(84) Etats contractants désignés :
AT BE CH DE ES FR GB IT LI NL SE

(71) Demandeur : **Espinasse, Françoise**
**44, rue de Tocqueville**
**F-75017 Paris (FR)**

(72) Inventeur : **Espinasse, Françoise**
**44, rue de Tocqueville**
**F-75017 Paris (FR)**

(74) Mandataire : **Wagret, Jean-Michel**
**23 rue de Léningrad**
**F-75008 Paris (FR)**

(54) **Ensemble d'électrodes destiné à l'établissement d'un électrocardiogramme.**

(57)    Un dispositif pour la prise d'une tension électrique sur le corps humain notamment à des fins de diagnostic médical comprend un ensemble d'électrodes destinées à être raccordées à un appareil de mesures et d'enregistrements tel qu'un électrocardiographe (E). Le dispositif comporte quatre électrodes dont trois électrodes de mesures (BD,BG etJG), sont raccordées à des membres latéraux opposés (supérieurs ou inférieurs) et à un troisième membre. La quatrième électrode (N), électrode de masse, également raccordée à l'appareil de mesure (E) permet d'éliminer les parasites et de stabiliser les mesures et leurs représentations graphiques. Les électrodes de mesures (BD,BG) sont chacune constituées par une armature support comportant des branches en matériau isolant (1,2), munies de plaques conductrices (5). Les palques conductrices (5) de la troisième électrode de mesure (JG) et de l'électrode de masse (N) sont montées sur les branches (1,2) d'une même armature support et sont raccordées chacune par une liaison filaire indépendante (8) à une prise spécifique de l'appareil de mesure (E). L'armature support des deux électrodes (JG,N) est apte à être positionnée sur le même membre (inférieur ou supérieur). La mise en place du système d'électrodes ne nécessite ainsi que la manipulation de trois supports au lieu de quatre.

EP 0 442 774 A1

FIG 2

La présente invention concerne un dispositif d'électrodes ou ensemble d'électrodes destiné à la prise d'une tension électrique notamment à usage médical et plus spécialement pour l'établissement d'un électrocardiogramme.

On sait que le travail du muscle cardiaque détermine un champ électrique susceptible d'être détecté sur le corps et les variations de tension caractéristiques de ce champ électrique, après amplification, permettent de fournir des enseignements sur le fonctionnement et l'état de santé du système cardiaque.

Le champ électrique doit être détecté à partir d'au moins trois points correspondant à une triangulation dont le centre est sensiblement occupé par le muscle cardiaque.

Pour cette raison on procède habituellement au positionnement d'électrodes constituant les organes de saisies des caractéristiques du champ électrique sur chacun des membres latéraux, soit supérieurs, soit inférieurs.

Le praticien a l'habitude d'utiliser une électrode à chaque membre pour enregistrer l'électrocardiogramme. Selon le principe du triangle d'Einthoven, les trois électrodes appelées ci-après par convention "électrodes de mesure" sont placées aux deux membres supérieurs et la troisième au membre inférieur gauche. Une quatrième électrode de masse, placée au membre inférieur droit, permet de stabiliser le tracé (Comité de l'électrocardiographie de l'American Heart Association). En fait, cette électrode neutre peut avoir une position indifférente.

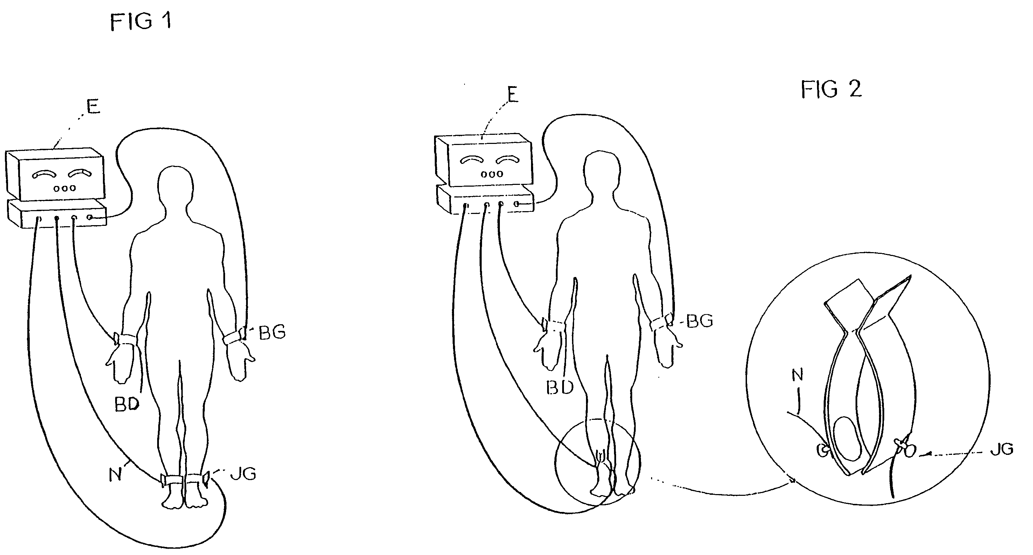

C'est cette méthodologie pratique qui est représentée à la Figure 1 et qui met en oeuvre trois électrodes triangulées de mesure respectivement aux deux bras et à la jambe gauche, plus une électrode de masse à la jambe droite permettant d'éliminer les parasites et de stabiliser le tracé. Les trois électrodes de mesure triangulées pourraient indifféremment être choisies en prenant les deux électrodes de jambe et une électrode de bras, la quatrième électrode de bras constituant alors l'électrode de masse.

Cette mise en place de deux électrodes respectivement droite et gauche sur les membres latéraux supérieurs et inférieurs, soit quatre électrodes au total, représente une certaine complexité dans la mesure où chaque électrode est reliée par un fil autonome à l'appareil de mesure et d'enregistrement électrocardiographique.

Or il a été constaté par la demanderesse qu'il était possible, après mise en place de deux électrodes actives sur deux membres latéraux opposés, d'obtenir des mesures permettant les saisies respectivement droite et gauche, à partir d'un seul troisième membre, en regroupant sur un même support une électrode active et une électrode de masse, chacune étant électriquement indépendante de l'autre et connectée par une liaison filaire indépendante à l'appareil de mesure.

Et dans ces conditions, la demanderesse a mis au point un dispositif d'électrodes apte à être mis en place sur un membre (inférieur ou supérieur) et comportant deux plaques conductrices, chacune connectée par une liaison électrique souple séparée à l'appareil de détection de mesure et d'enregistrement.

A cet effet, l'invention concerne un dispositif pour la prise d'une tension électrique sur le corps humain notamment à des fins de diagnostic médical et constituant un ensemble d'électrodes, ces électrodes étant destinées à être raccordées à un appareil de mesures et d'enregistrement tel qu'un électrocardiogramme et le dispositif étant du type comportant quatre électrodes dont trois électrodes de mesure, des caractéristiques d'un champ électrique, raccordées à des membres latéraux opposés (supérieurs ou inférieurs) et à un troisième membre, et une quatrième électrode, électrode de masse, également raccordée à l'appareil de mesures et permettant d'éliminer les parasites et de stabiliser les mesures et leurs représentations graphiques, deux des électrodes de mesure étant montées sur un support spécifique à chaque électrode, et le dispositif est caractérisé en ce que la troisième électrode de mesure et la quatrième électrode de masse sont montées sur une même armature support en matériau non conducteur, les deux électrodes étant indépendantes électriquement et raccordées chacune par une liaison filaire indépendante à une prise spécifique de l'appareil de mesures, l'armature support unique porteuse des deux électrodes étant apte à être positionnée sur le même membre (inférieur ou supérieur) la mise en place du système d'électrodes ne nécessitant ainsi que la manipulation de trois supports au lieu de quatre.

D'autres caractéristiques et avantages de l'invention ressortiront de la description qui suit et qui est donnée en rapport avec une forme de réalisation plus particulière présentée à titre d'exemple non limitatif en se référant aux dessins annexés dans lesquels

La Figure 1 représente un schéma de positionnement d'électrodes selon une technique classique.

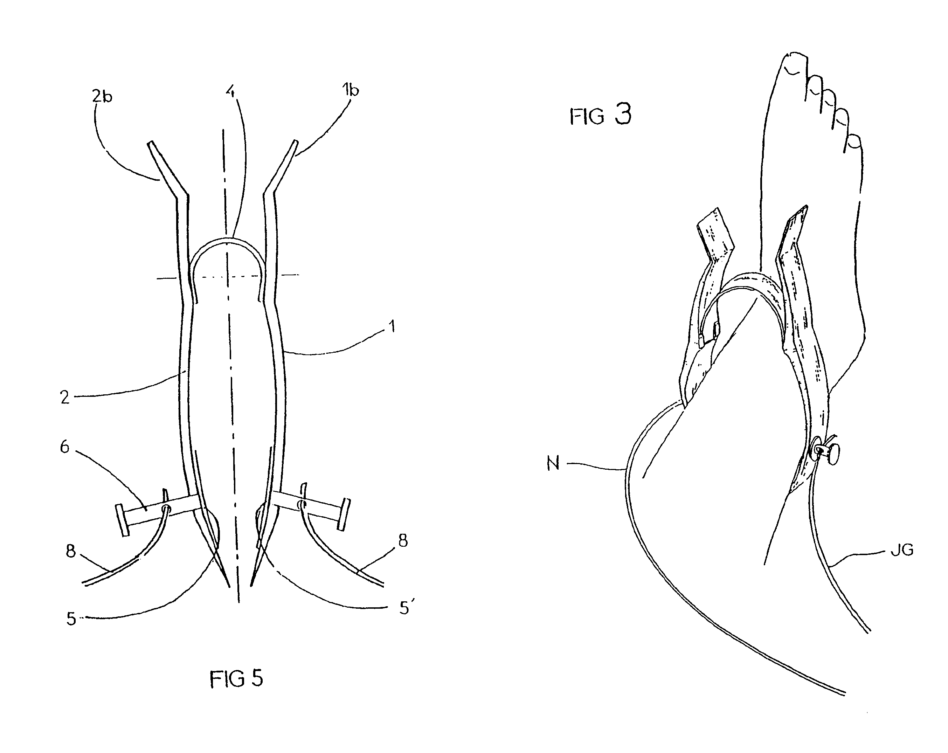

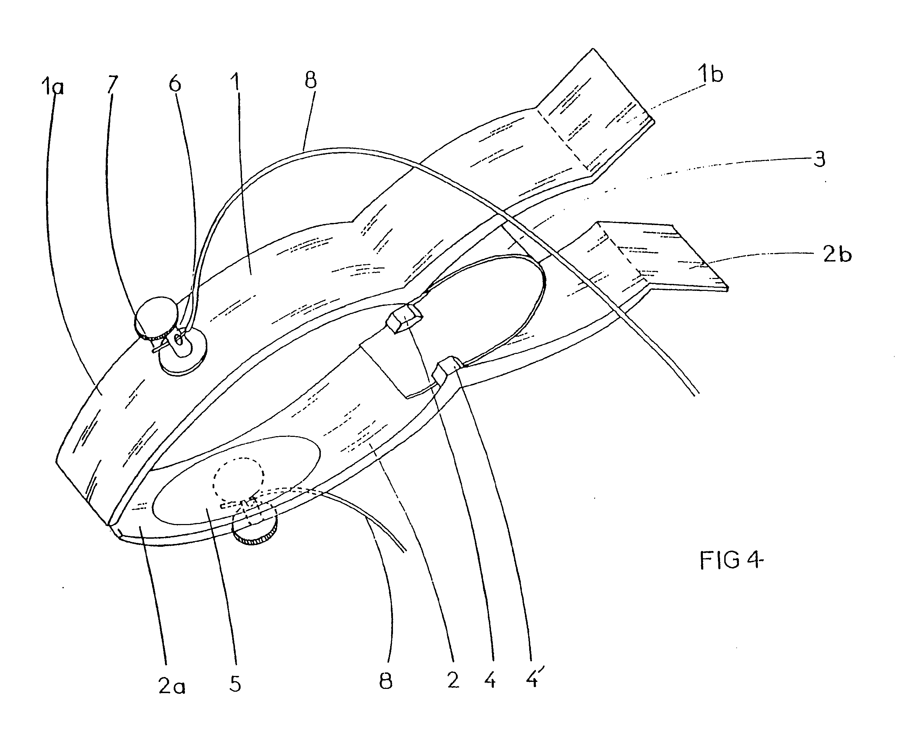

La Figure 2 représente un schéma de positionnement d'électrodes selon la présente invention en utilisant un support à deux électrodes tel que illustré aux Figures 3 et 4 et les Figures 1 et 2 montrant que l'électrocardiogramme dérivé des deux type de montage et de mesure est bien identique dans les deux cas.

La Figure 3 représente une vue en perspective de l'armature et des plaques conductrices selon l'invention à l'état nu.

La Figure 4 représente l'armature et le dispositif à deux électrodes mis en place sur le membre inférieur d'un patient.

La Figure 5 représente une vue en coupe du support à deux électrodes.

Selon l'ensemble des Figures, on voit que le dispositif d'électrodes est constitué de deux branches 1 et 2 indépendantes mais solidarisées entre elles par un organe intermédiaire 3 formant ressort du type "tube fendu".

Les extrémités des bras de ce ressort 3 du type "tube fendu" sont engagées et montées solidaires chacune respectivement de chacune des branches 1 et 2 de l'armature en exerçant une tension qui tend à ramener l'extrémité active respectivement 1a et 2a de chacune des branches l'une vers l'autre.

Les branches terminales du ressort 3 en "tube fendu" sont solidaires de chacune des branches 1 et 2, étant engagées dans des encoches respectivement 4 et 4' venues de chacune des branches.

Sur sa partie active 1a et 2a chacune des branches comporte sur sa face interne, faisant face à l'autre branche, une plaque en matériau conducteur par exemple en métal 5.

Cette plaque est solidaire d'un plot 6 qui traverse la paroi de chacune des branches et qui permet la réception de l'extrémité dénudée 7 d'un fil souple 8 engagé dans le plot et dont il est maintenu solidaire par un organe de pression physique telle par exemple une vis de pression manoeuvrée par la molette 9.

Chaque branche 1 et 2 comporte sa plaque 5 (seule la plaque 5 de la branche 2 étant visible sur la Figure 1).

Par ailleurs, on voit que la partie active 1a ou 2a de chacune des branches suit un profil bombé et incurvé, dont la concavité est orientée vers l'autre branche ce qui permet par conséquent à l'ensemble branches et plaques conductrices de venir épouser le contour bombé des faces, par exemple latérales, du membre inférieur récepteur du dispositif.

On voit que sur l'extrémité terminale 1b et 2b de chacune des branches 1 et 2 (chacune opposée à une partie active 1a, 2a) on a prévu un prolongement qui est orienté vers l'extérieur par rapport à l'axe médian de l'appareil, les deux prolongements 1b et 2b déportés angulairement vers l'extérieur constituent des moyens de préhension permettant de saisir l'ensemble formant pince, entre pouce et index, en provoquant ainsi par un seul mouvement l'écartement des deux branches 1 et 2 notamment de leur extrémité active 1a et 2a porteuse des plaques 5.

Il est alors aisé par un seul mouvement de positionner l'ensemble sur le membre récepteur.

Ce dispositif simplifie considérablement la mise en place des moyens de saisies des paramètres du champ électrique observés puisqu'un seul ensemble d'électrodes à pince peut par un mouvement très simple être mis en place sur le membre récepteur.

Les deux fils 8 peuvent bien entendu être conjugués et être réunis dans une seule gaine de façon à éviter tout problème de branchement et toute complication dans la mise en place.

Selon une disposition avantageuse, les deux branches respectivement 1 et 2, qui sont réalisées en matière synthétique, pourraient être prévues avec des caractéristiques visuelles différentes, par exemple chacune étant d'une couleur différente de l'autre; ce qui permet un repérage commode pour le praticien en vue du positionnement respectivement droite/gauche de chacune des plaques de prise de tension électriques.

L'intérêt du dispositif apparaît à la comparaison des Figures 1 et 2.

Alors que dans la Figure 1, correspondant à la mise en place traditionnelle, quatre électrodes disposées chacune sur un support, soit quatre supports, sont nécessaires et doivent être positionnées, dans l'illustration de la mise en oeuvre de l'invention, telle qu'apparaissant à la Figure 2, deux électrodes actives, chacune sur un support indépendant, restent positionnées sur chacun des membres supérieurs, tandis qu'une troisième électrode active et la quatrième électrode de masse (indépendantes électriquement l'une de l'autre) sont regroupées sur le même et unique support mis en place sur un seul membre inférieur.

De sorte que trois prises (et non quatre comme dans la méthode classique) seulement sont nécessaires et suffisantes, simplifiant le travail de l'assistant, évitant les erreurs de branchement et faisant gagner de précieuses minutes notamment en cas d'urgence cardio-vasculaire.

On comprend que le montage de la Figure 2 pourrait être transposé dans les mêmes conditions et avec le même résultat en plaquant deux électrodes actives aux membres supérieurs, une troisième électrode active sur un membre inférieur, cette troisième électrode active étant regroupée selon l'invention avec la quatrième électrode de masse, sur un même et unique support soit au membre inférieur soit au membre supérieur.

## Revendications

1. - Dispositif pour la prise d'une tension électrique sur le corps humain notamment à des fins de diagnostic médical et constituant un ensemble d'éléctrodes, ces électrodes étant destinées à être raccordées à un appareil de mesures et d'enregistrements tel qu'un électrocardiogramme (E) et le dispositif étant du type comportant quatre électrodes dont trois électrodes de mesures (BD, BG et JG), des caractéristiques d'un champ électrique, raccordées à des membres latéraux opposés (supérieurs ou inférieurs) et à un troisième membre, et une quatrième électrode (N) électrode de masse également raccordée à l'appareil de mesures et permettant d'éliminer les parasites et de stabiliser les mesures et leurs représentations graphiques, deux des électrodes de mesures (BD, BG) étant montées sur un support spécifique à chaque électrode, et le dispositif est caractérisé en ce que la troisième électrode de mesures (JG) et la quatrième électrode de masse (N) sont montées sur une même armature support en matériau non conducteur, les deux électrodes étant indépendantes électriquement et raccordées chacune par une liaison filaire indépendante à une prise spécifique de l'appareil de mesures (E), l'armature support unique porteuse des deux électrodes étant apte à être positionnée sur le même membre (inférieur ou supérieur) la mise en place du système d'électrodes ne nécessitant ainsi que la manipulation de trois supports au lieu de quatre.

2. - Armature support d'électrodes pour la prise d'une tension électrique sur le corps, notamment à usage médical et du type dans lequel l'armature est réalisée en matériau non conducteur et elle comporte deux branches (1, 2) d'écartement variable et solidaires entre elles, caractérisée en ce que chacune des branches est porteuse d'une plaque conductrice (5, 5') apte à venir au contact d'une des deux faces diamétralement opposée dudit membre, les deux plaques étant électriquement isolées l'une par rapport à l'autre et étant reliée chacune par une liaison filaire indépendante à l'appareil de mesures (E).

3. - Dispositif selon la revendication 2, caractérisé en ce que chaque branche comporte une face tournée vers l'autre épousant un profil curviforme concave et chacune des branches comporte sur cette face concave une plaque électriquement conductrice (5, 5'), chacune des plaques conductrices (5, 5') situées sur la face interne d'une branche étant en contact électrique par un plot traversant la paroi de ladite branche est apte à recevoir une connexion électrique d'un fil souple conducteur (8), spécifique à ladite plaque dont l'extrémité dénudée est maintenue sous pression dans ledit plot, en assurant ainsi la continuité électrique entre la plaque conductrice et le fil conducteur aboutissant à l'appareil de mesures (E).

4. - Dispositif selon l'une quelconque des revendication 2 ou 3, caractérisé en ce que les branches comportent un organe ressort du type "tube fendu" (3) assurant la solidarisation entre les branches et le rappel des deux branches vers leur position rapprochée, chaque extrémité du ressort étant solidaire de chaque branche, et le tube fendu (3) est réalisé en matière synthétique, ces extrémités libres étant engagées dans des encoches receptrices venues du moulage des branches elles-mêmes en matière synthétique, l'ensemble de l'armature support, porteuse des deux plaques constituant chacune une électrode indépendant, étant ainsi totalement dépourvue d'organemétallique.

5. - Armature support selon l'une quelconque des revendications 2 à 4, caractérisée en ce que les deux branches respectivement (1 et 2) sont réalisées en matière synthétique prévue avec des caractéristiques notamment de couleurs différentes en permettant un repérage commode pour le praticien en vue du positionnement convenable de l'armure support sur le membre afin de cette façon d'assurer le positionnement de chaque électrode de façon convenable par rapport audit membre.

FIG 1

FIG 2

6

FIG 3

FIG 5

FIG 4

EP 0 442 774 A1

Office européen **RAPPORT DE RECHERCHE EUROPEENNE** Numéro de la demande
des brevets

EP 91 40 0239

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| Y | US-A-3971366 (H. MOTOYAMA) <br> * colonne 5, ligne 9 - colonne 6, ligne 31 * | 1 | A61B5/0416 <br> A61B5/0428 |
| X | * figures 1a, 2d-2f * | 2, 3 | |
| Y | US-A-3826246 (W.J. RADDI ETAL.) <br> * colonne 4, ligne 8 - colonne 5, ligne 51 * | 1 | |
| X | * figures 3-6 * | 2, 3 | |
| A | GB-A-2133884 (CHILTERN INTERNATIONAL LTD.) <br> * page 2, lignes 1 - 23; figure 1 * | 1-4 | |
| A | US-A-3323516 (R.F. SALTER) <br> * colonne 2, ligne 24 - colonne 3, ligne 31 * | 3, 4 | |
| A | GB-A-2027292 (BUNKER RAMO CORP.) <br> * page 1, ligne 100 - page 2, ligne 38 * | 5 | |
| A | EP-A-157979 (FUKUDA DENSHI CO., LTD.) <br> * page 5, lignes 4 - 23; revendications 1-3; figures 2, 3 * | 1-3 | |
| | | | **DOMAINES TECHNIQUES RECHERCHES (Int. Cl.5)** |
| A | GB-A-2020981 (M. MOWBRAY) <br> * le document en entier * | 1, 2, 4 | A61B |

Le présent rapport a été établi pour toutes les revendications

| Lieu de la recherche | Date d'achèvement de la recherche | Examinateur |
|---|---|---|
| LA HAYE | 25 AVRIL 1991 | RIEB K.D. |

CATEGORIE DES DOCUMENTS CITES
X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant